# EUROPEAN PATENT SPECIFICATION

(11) **EP 2 794 912 B1**
(45) Date of publication and mention of the grant of the patent: **01.11.2017**
(21) Application number: 12806467.2
(22) Date of filing: 20.12.2012
(51) Int. Cl.: C12Q 1/68

(54) **METHODS RELATING TO IDIOPATHIC PULMONARY FIBROSIS (IPF)**
VERFAHREN IM ZUSAMMENHANG MIT IDIOPATHISCHER LUNGENFIBROSE (IPF)
PROCÉDÉS ASSOCIÉS À LA FIBROSE PULMONAIRE IDIOPATHIQUE (IPF)

(30) Priority: 21.12.2011 US 201161578339 P
(43) Date of publication of application: 29.10.2014
(73) Proprietor: The Provost, Fellows, Foundation Scholars, & the other members of Board, of the College of the Holy & Undiv. Trinity of Queen Elizabeth near Dublin, Dublin 2 (IE)
(72) Inventor: DONNELLY, Seamas, Dublin 2 (IE)
(74) Representative: FRKelly
(86) International application number: PCT/EP2012/076296
(87) International publication number: WO 2013/092810

(56) References cited:
- WO-A1-2011/094345
- WO-A2-00/60117
- C. T. RANJITH-KUMAR ET AL: "Effects of Single Nucleotide Polymorphisms on Toll-like Receptor 3 Activity and Expression in Cultured Cells", JOURNAL OF BIOLOGICAL CHEMISTRY, vol. 282, no. 24, 1 June 2007 (2007-06-01) , pages 17696-17705, XP055018029, ISSN: 0021-9258, DOI: 10.1074/jbc.M700209200
- P. ZHOU ET AL: "Toll-like receptor 3 C1234T may protect against geographic atrophy through decreased dsRNA binding capacity", THE FASEB JOURNAL, vol. 25, no. 10, 1 October 2011 (2011-10-01), pages 3489-3495, XP055050915, ISSN: 0892-6638, DOI: 10.1096/fj.11-189258
- MARGARITOPOULOS GIORGOS A ET AL: "Investigation of Toll-like receptors in the pathogenesis of fibrotic and granulomatous disorders: a bronchoalveolar lavage study", FIBROGENESIS & TISSUE REPAIR, BIOMED CENTRAL LTD, LONDON, UK, vol. 3, no. 1, 11 October 2010 (2010-10-11), page 20, XP021082898, ISSN: 1755-1536, DOI: 10.1186/1755-1536-3-20

## Description

The present invention concerns methods relating to idiopathic pulmonary fibrosis (IPF).

Idiopathic pulmonary fibrosis (IPF) is a progressive fatal lung disease characterized by persistent pulmonary inflammation.

It is not currently possible to predict the disease progression for patients presenting with IPF. Thus, patients with IPF have no knowledge of how aggressive the disease will be, nor do they have any insight into the most appropriate therapy for treating the disease. WO 00/60117 and WO 2011/094345 disclose SNPs (including variations in MUC5B, IL-1RN,TNF-A genes) which are associated with interstitial lung disease (ILD) and pulmonary diseases.

It is an object of the invention to overcome or mitigate the above-mentioned problems.

The invention is based on the finding that a sub-group of IPF patients carry a specific single nucleotide polymorphism (SNP) in one or both alleles of the toll-like receptor 3 (TLR3) gene. The SNP is identified as C1234T in human TLR3 mRNA (Reference SNP Cluster Report rs 3775291), which gives rise to the amino acid substitution L412F (i.e. leucine at position 412 replaced by phenylalanine) in the corresponding protein.

Applicant has found that patients who carry the C1234T polymorphism in both alleles of the TLR3 gene have a more aggressive clinical phenotype of IPF than patients who carry the C1234T polymorphism in one allele only or who do not carry the C1234T polymorphism in either allele of the TLR3 gene.

Patients who carry the C1234T polymorphism in both alleles may be referred to as TT homozygotes (Phe/Phe phenotype); patients who carry the C1234T polymorphism in one allele may be referred to as CT heterzygotes (Leu/Phe phenotype); and patients who do not carry the C1234T polymorphism in either allele (i.e. wildtype) may be referred to as CC homozygotes (Leu/Leu phenotype)

The invention provides a method of classifying a subject having idiopathic pulmonary fibrosis (IPF) based on genotype comprising the steps of:
a) providing a sample of a subject to be tested;
b) determining from the sample whether the subject:
   i) is homozygous (Phe/Phe) for the TLR3 gene/ C1234T/L412F polymorphism;
   ii) is heterozygous (Leu/Phe) for the TLR3 gene/C1234T/L412F polymorphism; or
   iii) is homozygous for the C allele (Leu/Leu) and
c) classifying the subject as belonging to a moderate phenotype of IPF or an aggressive phenotype of IPF, based on the genotype determined in b).

In accordance with the invention a subject is classified as belonging to a moderate phenotype of IPF based on the determination of the subject as being heterozygous (Leu/Phe) for the TLR3 (C1234T) L412F polymorphism or as having the wild type (Leu/Leu) TLR3 gene.

In accordance with the invention a subject is classified as belonging to an aggressive phenotype of IPF based on the determination of the subject as being homozygous (Phe/Phe) for the TLR3 (C1234T) L412F polymorphism.

The invention further provides the use of the C1234T polymorphism in the TLR3 gene of a patient having IPF for classifying the patient as belonging to a moderate phenotype of IPF or an aggressive phenotype of IPF.

The subject referred to herein is preferably human.

As used herein, the term "sample" is intended to mean any bodily fluid or tissue that enables the genotype of the subject to be determined. Suitable samples include but are not limited to tissue, cells, whole blood, unfiltered or filtered blood serum, saliva and semen. A preferred sample comprises lung tissue such as pulmonary fibroblasts, and especially primary human pulmonary fibroblasts.

As used herein, the term "moderate phenotype of IPF" is intended to be characterized by a life expectancy of more than three years, optionally more than four years, further optionally more than five years, from diagnosis of IPF.

As used herein, the term "aggressive phenotype of IPF" is intended to be characterized by a life expectancy of less than three years, optionally less than two years, from diagnosis of IPF.

It is known that a polymorphism exists in the gene C1234T (L412F in the protein) coding for toll-like receptor 3 (TLR3). Individuals can be classified as having the wild-type gene (CC corresponding to Leu/Leu phenotype), or as being homozygous (TT corresponding to Phe/Phe phenotype) for the polymorphism, or as being heterozygous (CT corresponding to Leu/Phe phenotype) for the polymorphism.

Methods for detecting the presence or absence of the C1234T polymorphism in the human TLR3 gene are well known to those skilled in the art and include DNA sequencing, single strand conformation polymorphism, restriction fragment length polymorphism and hybridisation analysis. Detection of the C1234T polymorphism may also be based on analysis of the TLR3 protein by e.g. mass spectrometry.

Classifying a subject based on genotype as described herein conveniently provides prognostic information. For example, as described above, it provides prognostic information such as whether a subject belongs to a moderate phenotype of IPF or an aggressive phenotype of IPF. Based on such prognostic information, certain predictive information is also conveniently provided, such as being able to foretell or forecast any aspect of the course of or progression of IPF, either in the absence or presence of treatment. Examples of predictive information include e.g. the average life expectancy of a subject having IPF and/or the likelihood that a subject will respond to a particular treatment or therapy. Therefore, by providing a reliable method of classifying patients with IPF according to their genotype, patients can be provided with an accurate forecast of their disease progression, and they can be guided on the most appropriate therapy based on the expected disease progression.

The following examples serve to illustrate the invention but it will be appreciated that the invention is not limited to these examples.

### EXAMPLE 1

### Human Genetics Studies in IPF

A United Kingdom IPF DNA collection (n=157) was screened for the prevalence of the specific TLR3 polymorphism (L412F). The UK IPF DNA cohort came from Sheffield University (n=66) and University of Edinburgh (n=91) and the prevalence of homozygotes was 12.1%. There were significantly higher numbers of homozygotes in the IPF cohort compared to in the control population (P < 0.05, Odd Ratio: 2.79). The results are shown in Table 1.

The Edinburgh cohort is an ongoing collection where maximal follow up is 2.5 years. Full mortality data is not yet available. The Sheffield University collection is from 11 years ago and full mortality data for all patients has been collected. The mean months from diagnosis to death for each genetic subgroup was found to be:-

| | |
|---|---|
| Wildtype: | 46.4 months +/- 5.5, |
| Heterozygote: | 52.8 months +/- 11.8, |
| Homozygote: | 20.6 months +/- 7.6. |

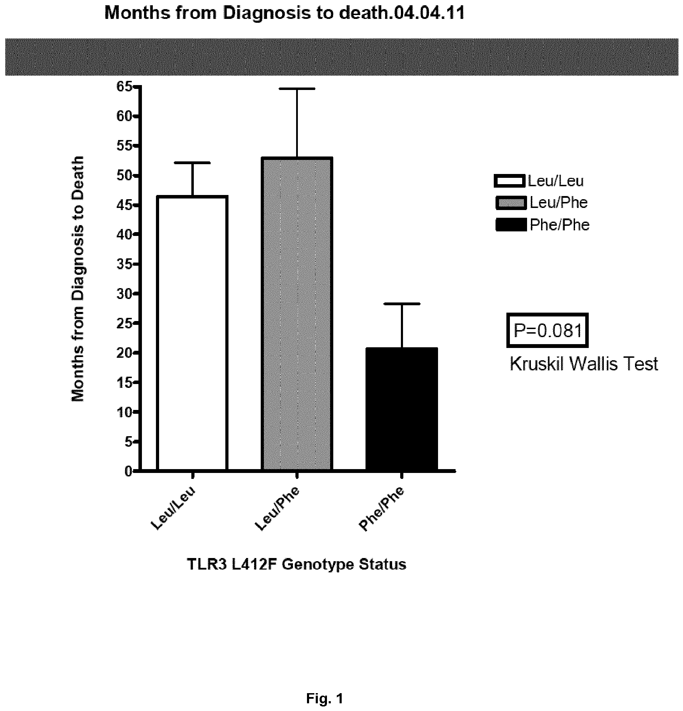

The mean months from diagnosis to death for each genetic subgroup is illustrated in Fig. 1.

**Table 1. TLR3 L412F polymorphism frequencies in UK IPF^{†} Cohort: Case-Control Study.**

| **Status** | **Leu/Leu** | **Leu/Phe** | **Phe/Phe** |
|---|---|---|---|
| **UK Controls** (n=158) | **75** *(0.47)* | **74** *(0.47)* | **9** *(0.06)* |
| **UK IPF Cases** (n=157) | **87** *(0.55)* | **51** *(0.33)* | **19** *(0.12)* |
| | | | |

| **P values** | | | |
|---|---|---|---|
| **Genotype** (using Chi-squared test for Independence) | | 0.013^{a} | |
| **Trend** (using Chi-squared test for Trend) | | 0.833 | |
| **Allele** (using Fisher's Exact Test) | | 0.860 | |
| | | | |

| **Calculation of Odds ratio (95% C.I.) using Fisher's Exact Test** | | | |
|---|---|---|---|
| **Phe carrier** | P value: **0.176** | 0.720 (0.466 - -1.133) | |
| **Phe/Phe homozygote** | P value: 0.049 | 2.279 (0.997 - 5.209) | |

| | | | |
|---|---|---|---|
| † Idiopathic Pulmonary Fibrosis diagnosed as per International Consensus Guidelines. American Thoracic Society. Idiopathic pulmonary fibrosis: diagnosis and treatment. International consensus statement. American Thoracic Society (ATS),and the European Respiratory Society (ERS). Am J Respir Crit Care Med, 2000 161(2 Pt 1). p. 646-64. | | | |

### EXAMPLE 2

### In Vitro Cellular Work in IPF

The inventors built up a biorepository of primary human pulmonary fibroblasts derived from patients with IPF. In relation to cells derived from IPF patients, the cells were screened for the specific TLR3 polymorphism (L412F). The homozygous cells (n=2 donors) were then compared with wildtype cells (n=2 donors) derived from IPF patients and a normal human pulmonary fibroblast cell line derived from the ATCC (CC19Lu) following Poly (I:C) stimulation. The results were noted, particularly with regards to readouts of:-
a) IRF3 pathway (Rantes production), and
b) NFkB pathway (IL-8 production).

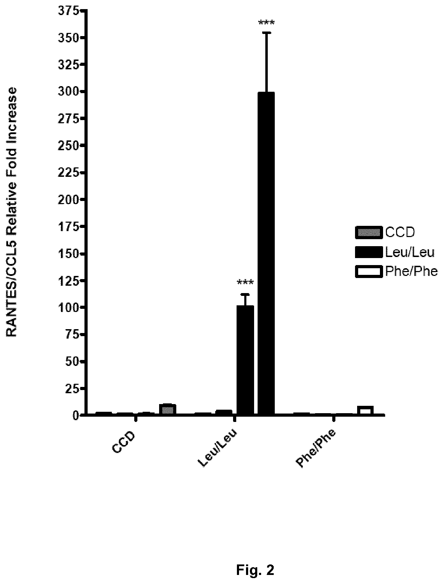

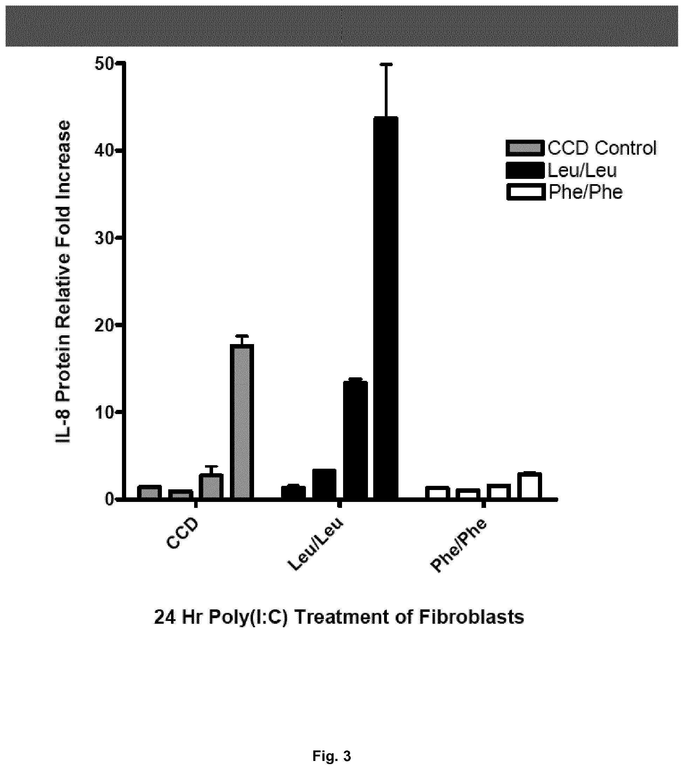

The results are shown in Figs. 2 and 3. It was determined that defective homozygous cells (Phe/Phe) have a blunted IRF3 & IL-8 response compared to wildtype IPF fibroblasts (Leu/Leu) and normal fibroblasts (CCD).

### EXAMPLE 3

### In-vivo data in Bleomycin Model - IPF

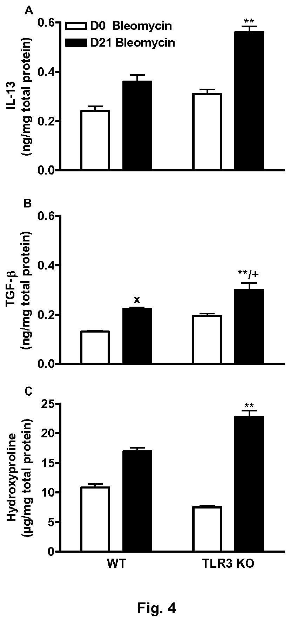

Experiments were performed using the murine Bleomycin pulmonary fibrosis model on both a TLR3 knockout and wildtype background. Experiments on the TLR3 -/- mice exhibited significantly enhanced pulmonary fibrosis as manifested by enhance IL-13, TGFB and hydroxyproline in lung homogenates. The results are shown in Fig. 4 from which the following may be observed:
TLR3 ⁻/⁻ mice experience increased pulmonary IL-13, TGF-β and collagen production compared with TLR3⁺/⁺ mice at day 21 (D21) post-bleomycin challenge compared with TLR3⁺/⁺ mice. **(A)** IL-13 and **(B)** TGF-β were significantly increased in lung homogenates in TLR3⁻/⁻ mice at D21 compared with day 0 (D0) post-bleomycin challenge as quantitated by ELISA **(B)** TGF-β was significantly increased in lung homogenates at D21 post-bleomycin challenge in TLR3⁻/⁻ mice compared with TLR3⁺/⁺ mice **(C)** Hydroxyproline levels were significantly increased in whole lung homogenates at D21 post-bleomycin challenge in TLR3⁻/⁻ mice, but not TLR3⁺/⁺ mice. **(A-C)** ***P* < 0.01, ****P* < 0.001: D21 TLR3⁻/⁻ compared with D0 TLR3⁻/⁻ mice. +p<0.05: D21 TLR3⁻/⁻ mice compared with D21 TLR3⁺/⁺ mice. ^{x}p<0.05: D21 TLR3⁺/⁺ compared with D0 mice.

## Claims

1. A method of classifying a subject having idiopathic pulmonary fibrosis (IPF) based on genotype comprising the steps of:
a) determining from a sample of a subject to be tested whether the subject:
i) is homozygous (Phe/Phe) for the TLR3 gene C1234T/L412F polymorphism;
ii) is heterozygous (Leu/Phe) for the TLR3 gene C1234T/L412F polymorphism; or
iii) is homozygous for the C allele (Leu/Leu) and
b) classifying the subject as having a moderate phenotype of IPF if the subject is heterozygous for the TLR3 gene C1234T/L412F polymorphism, or if the subject is homozygous for the C allele; and classifying the subject as having an aggressive phenotype of IPF, if the subject is homozygous for the TLR3 gene C1234T/L412F polymorphism.

2. A method according to claim 1, wherein step a) is carried out by detecting the presence or absence of the amino acid substitution L412F in the TLR3 protein.

3. A method according to any preceding claim, wherein the sample to be tested comprises human primary pulmonary fibroblasts.

4. A method according to any preceding claim, wherein the subject is human.

5. Use of the C1234T/L412F polymorphism in the TLR3 gene for classifying a patient having IPF as belonging to a moderate phenotype of IPF or an aggressive phenotype of IPF, wherein the patient is classified as belonging to a moderate phenotype of IPF if heterozygous (Leu/Phe) for the TLR3 gene C1234T/L412F polymorphism or if homozygous for the C allele and wherein the patient is classified as belonging to an aggressive phenotype of IPF based on the determination of the patient if homozygous (Phe/Phe) for the TLR3 gene C1234T/L412F polymorphism.

## Patentansprüche

1. Verfahren zur Einstufung eines Subjekts mit idiopathischer Lungenfibrose (IPF) basierend auf einem Genotyp, das die folgenden Schritte umfasst:
a) Bestimmen aus einer Probe eines zu testenden Subjekts, ob das Subjekt:
i) homozygot (Phe/Phe) für den TLR3-Gen-C1234T/L412F-Polymorphismus ist;
ii) heterozygot (Leu/Phe) für den TLR3-Gen-C1234T/L412F-Polymorphismus ist; oder
iii) homozygot für das C-Allel (Leu/Leu) ist, und
b) Einstufen des Subjekts, ob es einen moderaten Phänotyp von IPF aufweist, wenn das Subjekt für den TLR3-Gen-C1234T/L412F-Polymorphismus heterozygot ist oder wenn das Subjekt für das C-Allel homozygot ist;
und Einstufen des Subjekts, ob es einen aggressiven Phänotyp von IPF aufweist, wenn das Subjekt für den TLR3-Gen-C1234T/L412F-Polymorphismus homozygot ist.

2. Verfahren nach Anspruch 1, wobei Schritt a) durch den Nachweis der Gegenwart oder Abwesenheit der Aminosäuresubstitution L412F in dem TLR3-Protein durchgeführt wird.

3. Verfahren nach einem vorstehenden Anspruch, wobei die zu testende Probe menschliche primäre pulmonale Fibroblasten umfasst.

4. Verfahren nach einem vorstehenden Anspruch, wobei das Subjekt ein Mensch ist.

5. Verwendung des C1234T/L412F-Polymorphismus in dem TLR3-Gen zur Einstufung eines Patienten mit IPF, ob er zu einem moderaten Phänotyp von IPF oder einem aggressiven Phänotyp von IPF gehört, wobei der Patient als zugehörig zu einem moderaten Phänotyp von IPF eingestuft wird, wenn er für den TLR3-Gen-C1234T/L412F-Polymorphismus heterozygot (Leu/Phe) ist oder wenn er für das C-Allel homozygot ist, und wobei der Patient als zugehörig zu einem aggressiven Phänotyp von IPF eingestuft wird, was auf der Bestimmung des Patienten basiert, wenn er für den TLR3-Gen-C1234T/L412F-Polymorphismus homozygot (Phe/Phe) ist.

## Revendications

1. Procédé de classification d'un sujet atteint de fibrose pulmonaire idiopathique (FPI) basé sur un génotype comprenant les étapes consistant à :
a) déterminer à partir d'un échantillon d'un sujet soumis à un test si le sujet :
i) est homozygote (Phe/Phe) pour le polymorphisme C1234T/L412F du gène du TLR3 ;
ii) est hétérozygote (Leu/Phe) pour le polymorphisme C1234T/L412F du gène du TLR3 ; ou
iii) est homozygote pour l'allèle C (Leu/Leu), et
b) classer le sujet comme ayant un phénotype modéré de FBI si le sujet est hétérozygote pour le polymorphisme C1234T/L412F du gène du TLR3 ou si le sujet est homozygote pour l'allèle C ; et classer le sujet comme ayant un phénotype agressif de FPI, si le sujet est homozygote pour le polymorphisme C1234T/L412F du gène du TLR3.

2. Procédé selon la revendication 1, dans lequel l'étape a) est réalisée en détectant la présence ou l'absence de la substitution d'acide aminé L412F dans la protéine TLR3.

3. Procédé selon l'une quelconque des revendications précédentes, dans lequel l'échantillon soumis à un test comprend des fibroblastes pulmonaires primaires humains.

4. Procédé selon l'une quelconque des revendications précédentes, dans lequel le sujet est humain.

5. Utilisation du polymorphisme C1234T/L412F du gène du TLR3 pour classer un patient atteint de FPI comme appartenant à un phénotype modéré de FPI ou un phénotype agressif de FPI, dans lequel le patient est classé comme appartenant à un phénotype modéré de FPI s'il est hétérozygote (Leu/Phe) pour le polymorphisme C1234T/L412F du gène du TLR3 ou s'il est homozygote pour l'allèle C et dans lequel le patient est classé comme appartenant à un phénotype agressif de FPI sur la base de la détermination que le patient est homozygote (Phe/Phe) pour le polymorphisme C1234T/L412F du gène du TLR3.
